# EUROPEAN PATENT SPECIFICATION

(11) **EP 1 942 971 B1**
(45) Date of publication and mention of the grant of the patent: **29.06.2011**
(21) Application number: 06802662.4
(22) Date of filing: 30.08.2006
(51) Int. Cl.: A61M 25/00, A61M 25/06, A61M 27/00, A61F 2/06, A61M 29/00

(54) **Catheter for implantation of Stents**
Katheter für Implantation von Stents
Cathéter pour implantation des Stents

(30) Priority: 31.08.2005 US 713151 P; 31.08.2005 US 218210
(43) Date of publication of application: 16.07.2008
(73) Proprietor: Cook Medical Technologies LLC, Bloomington, IN 47404 (US)
(72) Inventor: LAVELLE, Shay, County Limerick (IE)
(74) Representative: Garratt, Peter Douglas
(86) International application number: PCT/US2006/033944
(87) International publication number: WO 2007/027830

(56) References cited:
- EP-A1- 0 365 269
- US-A- 4 931 037
- US-A1- 2001 018 574

## Description

### TECHNICAL FIELD

The technical field of the invention is implantable medical devices, and in particular, medical devices which can be used in a technique for more readily dilating a body passage into which an access device or a stent is to be placed.

### BACKGROUND

Minimally-invasive surgery has evolved to a point where procedures that were unimaginable a few years ago are now routinely performed on a daily basis. Even in these procedures, however, there is room for improvement. One example is the removal of stones and calculi from kidneys and ureters, to the great relief of many suffering patients.

To treat this condition, several individual steps are involved. In one procedure, these steps include placing a relatively narrow guidewire through a urethra and a bladder, and then through the ureter and into the kidney. After the guidewire is placed, a catheter is run along the guidewire, dilating the body passages (the urethra and the ureter) as it moves down the guidewire. In the next sequence for this procedure, a ureteral access sheath is guided along and down the guidewire and the catheter. The access sheath also dilates the body passages as it moves from outside the body, through the urethra, and into the ureter, down to the desired location, and into or very near the kidney.

The physician may then remove calculi and stones through the access sheath using a grasper, a retrieval basket, or other device. The access sheath protects the ureter from repeated passage of the retrieval device while the stones or calculi are removed. After the stones are removed, a ureteral stent may be placed into the ureter through the access sheath, using the catheter or a pushing tube to position the stent. The stent is used to retain patency of the ureteral lumen and to continue normal urinary drainage.

One problem with this procedure is that the guidewire may need to be very long in order for the physician to control passage first of the catheter and then of the access sheath to the desired location within the patient's body. Very long guidewires are not standard, and it may require two people to handle such a guide wire so that it does not drape onto the floor. The surgeon may decide he or she needs a guidewire with a stiffness different from the one provided with the particular kit in order to negotiate the pathway. A substitute stiffer guidewire may not be readily available in non-standard lengths.

Using this procedure for sequential placement of first a catheter and then an access sheath, the guidewire needs to be as long as the combination of both the catheter and the access sheath. A long guidewire leads to two problems, including a greater tendency to kink and a need for greater skill on the part of the physician to maneuver the guidewire while placing the guidewire itself, the catheter, and the sheath.

Another problem that is encountered with ureteral stents occurs in cancer patients, where a growth may apply radial compression to a ureter. Such compression can make fluid flow difficult. In these cases, a typical polymeric, relatively soft pigtail stent may not have sufficient radial strength to resist compression by a cancerous or other growth. In these cases, a stronger, sturdier ureteral stent is needed to resist radial compression and allow for continued drainage from the kidney to the bladder. In some cases, a urethral stent or catheter may also be helpful to ensure drainage from the bladder.

Reference is directed to EP 0 365 269 in which there is provided an indwelling ureteral stent positioning apparatus is provided for allowing single intrusion placement of a stent in the ureter of a patient. The apparatus is pre-assembled and includes a wire guide with floppy tip. The stent is positioned about the wire guide adjacent to the floppy tip of the wire guide, and is adjustably situated on the wire guide to expose a pre-determined length of the floppy tip or insertion into the patient. The stent is a dual open-ended tubular stent having a resilient retentive curved circular or pigtail shape at each end for retention in the kidney and bladder. A positioner is conveyed on the wire stent until the end of the positioner abuts the anatomically distal end of the stent. A pin-vise clamp abuts the external end of the positioner and releasably engages the wire guide to stabilize the apparatus during insertion. When used to position the stent within the ureter, the apparatus is conveyed along the ureter until the floppy tip is within the renal collecting system. The positioner is advanced along the wire guide to push the anatomically proximal end of the stent over and beyond the floppy tip so that the retentive coil forms within the kidney. The pin-vise clamp is released to allow the wire guide to be withdrawn until it passes the anatomically distal end of the stent, wherein the retentive coil shape is formed within the bladder. With the stent fully indwelling, the positioner and wire guide are removed from the patient.
What is needed is a better way to dilate the body passages in order to place the access sheaths and stents.

### BRIEF SUMMARY

This need is met by embodiments of the present invention which is set forth in the appended claims. There is here disclosed a method including steps of placing a guidewire along a body path to a location desired for the stent, inserting an access sheath secured to a catheter along the guidewire, wherein the catheter and access sheath are substantially coaxial, and advancing the access sheath and catheter to the desired location, removing the guidewire, removing the catheter, advancing the stent within the sheath to the desired location using a stent positioner, the catheter optionally serving as the stent positioner. The sheath is then removed, leaving the stent.

There is also disclosed a method including providing a stent having closed distal and proximal ends, advancing a wire guide along a body path, inserting an access sheath secured to a catheter along the guidewire to a desired location within a patient, wherein the catheter and access sheath are substantially coaxial. The method also includes removing the guidewire, removing the catheter, advancing the stent within the sheath to the desired location using a stent positioner, the catheter optionally serving as the stent positioner, and removing the sheath.

The kit of the invention is defined in claim 1. There is further disclosed an access kit that includes a stent, a catheter having a proximal portion and a distal portion, and an access sheath having a proximal portion and a distal portion. The catheter is removably connected to the access sheath. The catheter and the access sheath are substantially coaxial. The catheter is longer than the access sheath, and the length of the catheter is less than twice the length of the access sheath.

There are many ways to practice the present invention, of which the drawings and description below depict only a few.

### BRIEF DESCRIPTION OF THE DRAWINGS

The embodiments will be further described in connection with the attached drawing figures. It is intended that the drawings included, as a part of this specification, be illustrative of the embodiments and should in no way be considered as a limitation on the scope of the invention.

Fig. 1 is an illustration of a present technique for ureteral stent placement;

Fig. 2 is an illustration of a technique for dual dilatation;

Fig. 3 is a cross-sectional view of a first embodiment of a kit according to the present invention;

Figs. 3a and 3b depict a catheter and a sheath useful in kit embodiments;

Fig. 4 depicts a pigtail ureteral stent;

Figs. 5, 5a, and 5b depict a ureteral stent useful in kit embodiments;

Fig. 5c depicts an alternate embodiment of the stent;

Fig. 6 depicts a stricture in a body lumen;

Fig. 7 is a catheter configured as a rapid exchange system;

Fig. 8 is a catheter configured as a rapid exchange system; and

Fig. 9 is a catheter configured as a rapid exchange system.

### DETAILED DESCRIPTION OF THE DRAWINGS AND THE PRESENTLY PREFERRED EMBODIMENTS

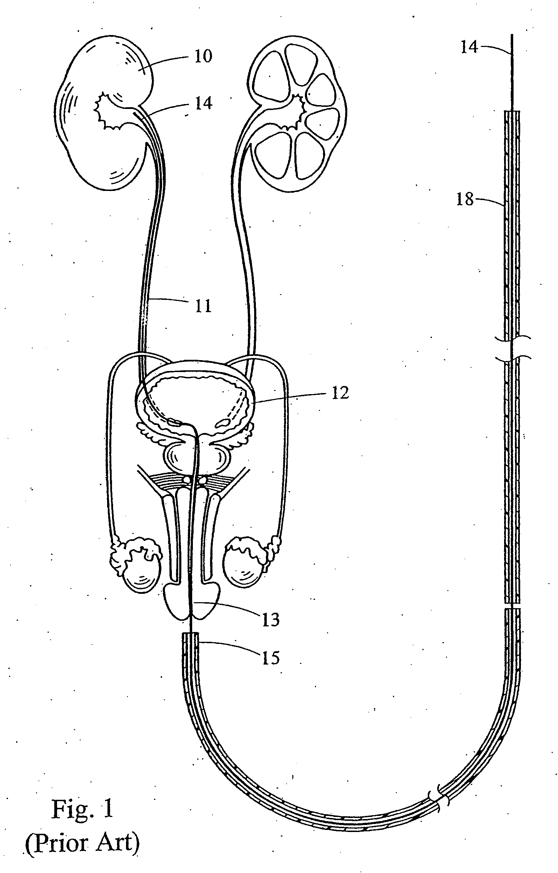

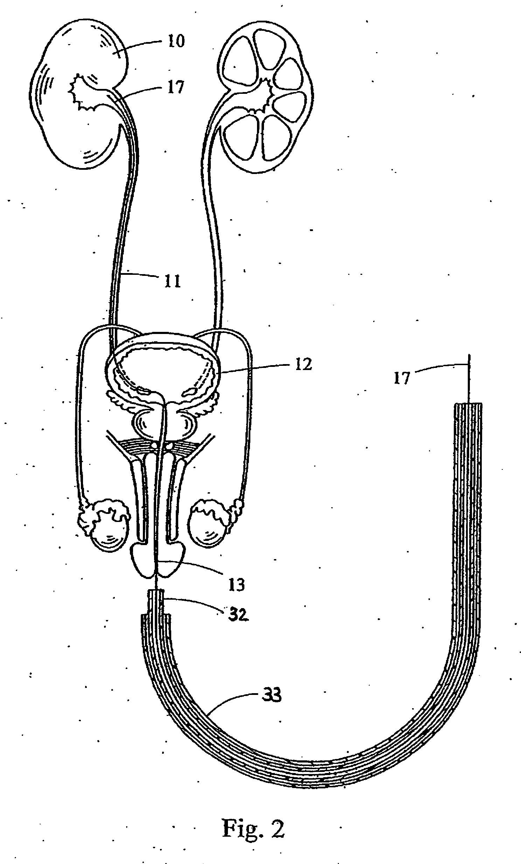

Figs. 1 and 2 illustrate the differences in technique between a present method of ureteral stent placement and a new method of coaxial dual dilatation. In both figures, a physician desires to perform a procedure upon a kidney 10. In Fig. 1, a guidewire 14 is advanced through a urethra 13, a bladder 12, and a ureter 11 to the kidney on which the procedure is to be performed. In order to accomplish this, the wire guide is placed, and a ureteral stent 15 is guided along the guidewire, extending as far as desired, typically into the kidney by means of a pushing tube 18 that is also placed along the guidewire as shown. The physician places the stent by passing first the guidewire, and then passing the ureteral stent and the pushing tube over the guidewire. The urethra may be dilated separately to accommodate an instrument such as a cystoscope to aid the surgeon.

The guidewire is typically between 0.018 to 0.038 inches in diameter (about 0.46 mm to 0.97 mm). The catheter may be 4-8 Fr (I French=0.3mm). The ureteral stent may be used for patency of the ureteral lumen. In order to achieve this dilatation, however, a very long wire guide was needed to extend the length of both the catheter and the access sheath, where the access sheath is capable of extending to the ureteropelvic junction. This may lead to kinking and may also lead to difficulty in the physician controlling the wire guide as he or she must control the entire length of the wire guide while sequentially running the catheter and the access sheath down the wire guide.

An improved method is illustrated in Fig. 2. In this method, a physician places a wire guide 17 through a urethra 13, a bladder 12, and a ureter 11 into a kidney 10. After the wire guide is placed, a catheter 19 secured to an access sheath 16 is guided along the wire guide, the catheter and access sheath combination coaxially "dual dilating" at least the proximal portion of the ureter. This coaxial dilatation procedure enables the physician to use a shorter wire guide, e.g., using a 145 or 150 cm wire guide rather than a wire guide that may have to be 220 cm or even longer, perhaps 250-260 cm. This may also shorten the time required to position the access sheath, and thus shorten the actual time spent in the therapeutic procedure and reduce the number of personnel required. The access sheath and the catheter are advanced to the desired location, e.g., into the calices of a kidney. The catheter may then be removed and replaced by a stent. The stent is then implanted by a surgeon pushing on a stent positioner, such as a catheter or other pushing device of appropriate diameter and length. Thus, the catheter itself may be retracted all or part way to allow positioning of the stent within the sheath, and the catheter re-placed to serve as the stent positioner. The sheath is then retracted while the positioner or other device is used to keep the stent in place.

In addition to the method described above and shown in Fig. 2, there are other ways to practice the invention. For instance, rather than accessing the ureter through the urethra and bladder, a physician may use a nephrostomy method, in which the access sheath and catheter are advanced through a person's skin to reach the calices of the kidney directly. If a path to a bile duct is needed, the physician may access the bile duct through an endoscope via the mouth, esophagus, stomach, and intestines, or via laparoscopic methods directly through the skin (percutaneous). If vascular access is desired, a physician may access the blood vessel through an opening, such as an opening manufactured in the femoral artery.

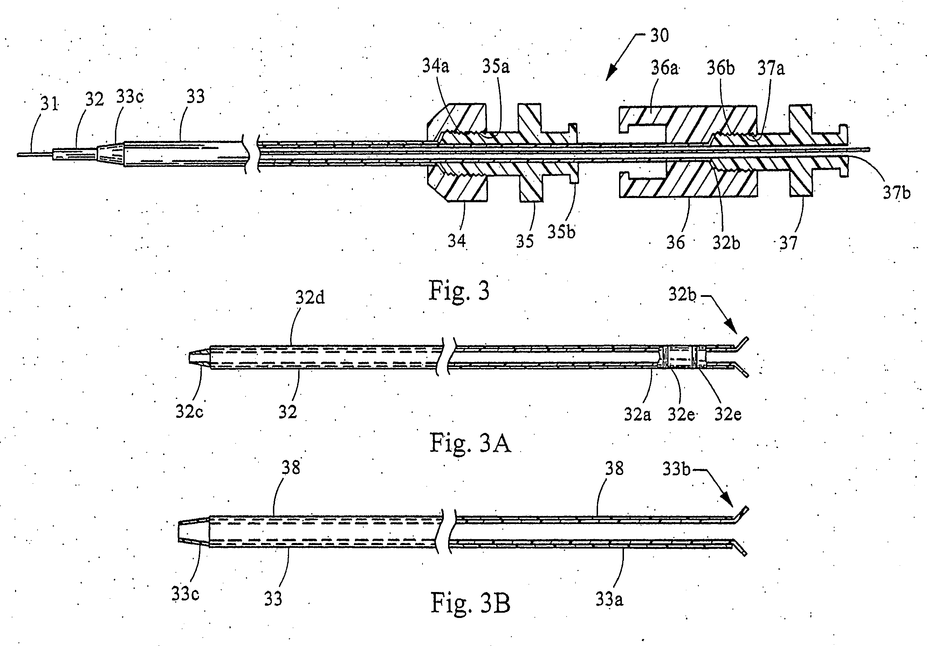

An embodiment of a kit useful in the above procedure is depicted in Figs. 3, 3a and 3b. The kit includes co-axial introducer 30 that includes wire guide 31, which may be shorter than a wire guide used for a sequential procedure as described above. A wire guide with a length of about 145-150 cm is preferred, but other lengths may be used. The stent positioner is often a catheter. A catheter 32 is included, the catheter preferably having a proximal end 32a with a flared tip 32b, and a soft rounded/tapered non-traumatic tip 32c for protection of the patient. Materials for the catheter are typically plastic or elastomeric materials, e.g., PVC, PTFE, polyurethane, silicone, and urethane, but any medically acceptable materials may be used. Catheters suitable for this use are about 50-85 cm long; however, longer or shorter catheters are contemplated. Suitable catheters have a diameter of about 6 Fr.; however, other diameters are contemplated depending upon the needs of the patient and the procedure to be performed. The tip is flared for ease in securing to connectors and in sealing with connectors so that the catheter may deliver a fluid, such as a radiopaque fluid for diagnostic procedures or for visualization purposes. The catheter may have a hydrophilic coating 32d on at least part of its outer surface. The proximal portion may also have one or more marking bands 32e to assist the physician in deploying the stent. In addition, marking bands made from a radiopaque material to be observable using x-ray, fluoroscopic, or other suitable detection means, can be placed on the distal portion of catheter 32 and access sheath 33 to increase visibility.

Catheter 32 may interface with one or more connectors 36 for mating with syringe adapter 37 (such as a female Luer lock adapter) so that a syringe (not shown) can inject the radiopaque fluid. Connector 36 may include a male Luer lock fitting 36a on a distal end of connector 36 and internal threads 36b on its proximal end. Male Luer lock connection 36a may be used to connect first connector 36 to second connector 35. Threads 36b may interface with matching external threads 37a of syringe adapter 37 for delivery of a fluid through lumen 37b. Flared tip 32b of the catheter helps to seal the connection between connector 36, catheter 32, and syringe 37. While the Luer lock and threaded connections depicted and described are preferred, other connectors may be used instead. For example, quick-release connectors could be used to secure the catheter or sheath to their proximal fittings. When connectors 36 and 37 are joined with flared tip 32b, a leak-tight connection is formed, and the catheter may reliably deliver fluid without undesirable leakage.

Access sheath 33 includes a proximal portion 33a and an end portion with a flared tip 33b. The access sheath also includes a distal end 33c, preferably atraumatic, soft and rounded or tapered for ease of introduction into the patient. Distal end 33c of the access sheath is also preferably more highly radiopaque than the remainder of the access sheath, so that the end may be observed with x-ray or fluoroscopic detection means during the implantation procedure. Flared tip 33b helps to seal an interface between access sheath 33 and connector 34. Access sheaths are preferably made from low friction polymers (e.g. PTFE, FEP, etc.) with reasonable radial compressive strength - wire reinforcement can be added to the sheath for extra radial strength. Suitable access sheaths sold under the name of Check-Flo® II Introducer sheaths sold by Cook Incorporated, Indiana may be used. Also, Flexor® sheaths available from Cook Urological Incorporated of Spencer, Indiana may be used. In this application the sheath is typically 70 cm long so to extend from the urethral meatus to the ureteropelvic junction. The access sheath is generally just slightly larger in inner diameter than the outer diameter of the catheter, e.g. 0.5 Fr. Suitable access sheaths have a diameter of about 6.5 Fr.; however, other diameters are contemplated depending upon the needs of the patient and the procedure to be performed. If catheter 32, as shown in Fig. 3 and preferably with a blunt distal tip, is the same size diameter as the stent, the catheter may be used as a stent positioner, with the physician simply butting the distal end of the catheter against the proximal end of the stent so that the positioner can be used to push the stent into position.

Connector 34 may include internal threads 34a for connecting to Luer lock connector 35 having external threads 35a and female Luer lock connection 35b. While Luer lock connections and connectors are preferred, other connectors and other types of medically-acceptable connectors may be used. At least a distal portion of sheath 33 may also include a hydrophilic coating 38.

The fittings described above may be used to connect access sheath 33 with catheter 32. To help insure that access sheath 33 seals securely, connector 34 may be temporarily joined to connector 35 with an adhesive. Other methods may also be used, such as securely tightening connectors 34, 35 together. Joining the female Luer lock connection 35b to male Luer lock connection 36a reliably secures access sheath 33 to catheter 32 for insertion or for removal. By breaking the connection between connectors 35,36 after insertion, catheter 32 may be removed and the access sheath may be used for other purposes. These other purposes may include diagnostic purposes, such as insertion of an endoscope, or therapeutic procedures, such as breaking up stones or calculi, using a holmium laser or other type of lithotripter. A grasper or basket may then be inserted into the working channel of the endoscope to remove the fragments. In the same manner, connectors 36, 37 may also be temporarily joined with an adhesive to prevent easily breaking the connection. By adhering connector pairs 34, 35 and 36, 37, it is easier for the surgeon to make and break the Luer lock connection between connectors 35, 36.

In the assembled view of Fig. 3, note that the catheter may be longer than the access sheath, and may extend slightly further distally than the access sheath. Nevertheless, the sheath and the catheter are substantially coaxial, i.e., catheter 32 runs the entire length of access sheath 33. Substantially coaxial means that substantially the length of one of the sheath and the catheter is coaxial with the other of the sheath and the catheter during the procedure for implanting a stent or other device into a human or mammalian body.

In addition, the catheter (and/or the access sheath) can be configured to be a "rapid exchange" system. A rapid exchange system, also known as a "short wire guide" or "monorail" system, is an alternative technique for guiding a delivery catheter to a target site in a patient body by utilizing catheters having a relatively short wire guide lumen. In such systems, the wire guide lumen extends only from a first lumen opening spaced a short distance from the distal end of the catheter to a second lumen opening at or near the distal end of the catheter. As a result, the only lumenal contact between the catheter's wire guide lumen and the wire guide itself is the relatively short distance between the first and second lumen openings. Several known advantages are conferred by this configuration. For example, the portion of the wire guide outside the patient's body may be significantly shorter than that needed for the "long wire" configuration. This is because only the wire guide lumen portion of the catheter is threaded onto the wire guide before directing the catheter through the desired path (e.g., a working lumen of the access sheath, etc.) to the target site.

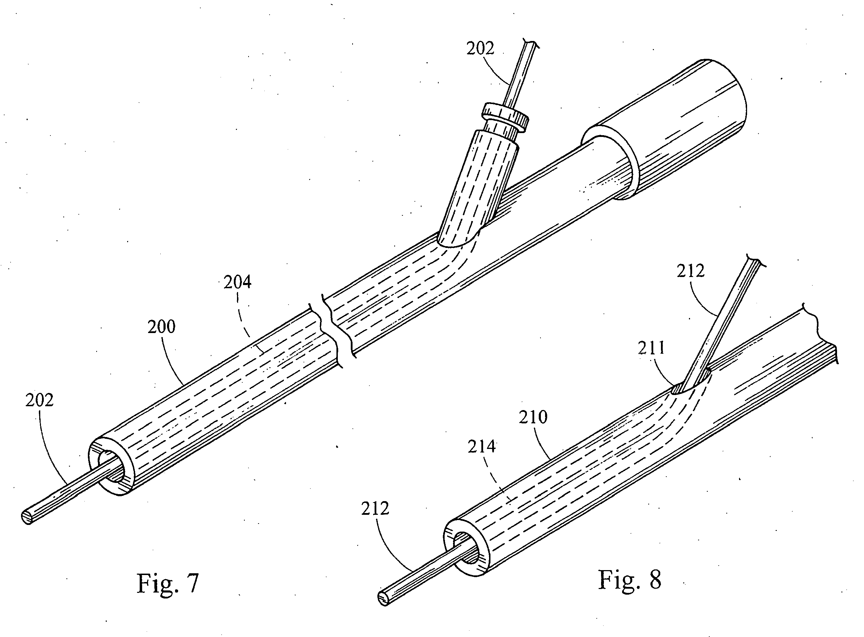

By way of illustration, Figs. 7 and 8 illustrate the distal ends of two different types of catheters. Fig. 7 shows the distal end of long-wire catheter shaft 200 with a wire guide 202 disposed in a lumen 204. The lumen 204 extends substantially to the proximal end of the catheter shaft 200. (Note: The wire guides illustrated throughout this specification are drawn to illustrate the concepts being described and may not be shown to scale; preferred wire guides typically have an external diameter that is nearly the same as the internal diameter of catheter lumens through which they are passed.)

Fig. 8 shows the distal end of short-wire catheter shaft 210 with a side port aperture 211 and wire guide 212 disposed in lumen 214. The length of lumen 214, and consequently the exchange length of catheter 210, is substantially shorter than that of catheter 200 shown in Fig. 7. In addition to a shorter exchange length, catheter 210 (Fig. 8) has a reduced surface contact between the wire guide and catheter lumen that results in a reduced friction between the two. This can result in an eased threading and exchange process by reducing the time and space needed for catheter exchange. This economy of time and space is advantageous for minimally invasive surgeries by reducing the likelihood of contamination and reducing the total time and stress of completing surgical procedures.

In certain rapid exchange catheter configurations, the wire guide lumen is open to a side port aperture in the side of the catheter between its proximal and distal ends. In one such configuration, the wire guide lumen only extends from the side port aperture to an opening at the distal end. An example of this type of rapid exchange catheter is illustrated in Fig. 8.

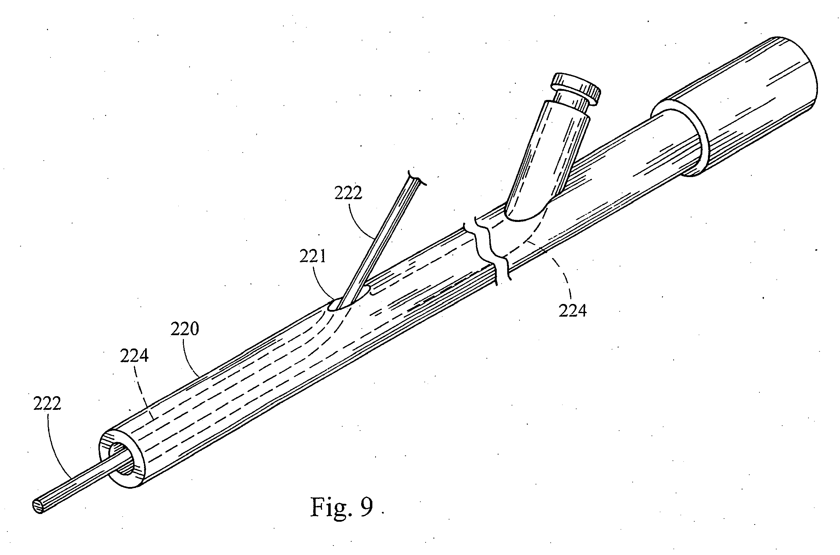

In another type of rapid exchange catheter configuration, the wire guide lumen extends through the length of the catheter from near its proximal end to its distal end. A side port aperture between the proximal and distal ends opens into the wire guide lumen. This side port aperture allows the catheter to be used in a short wire guide configuration, while the full-length wire guide lumen allows the catheter to be used in a long wire guide configuration. This wire guide lumen configuration is referred to as "convertible" or "dual use." An example of this type of catheter is illustrated in Fig. 9, which shows the distal end of "convertible" catheter shaft 220 with wire guide 222 disposed through a side port aperture 221 and into a wire guide lumen 224. Specifically, a wire guide may run through substantially the entire length of the wire guide lumen, or the wire guide may run only through the portion of the lumen between the distal end and the side port aperture. The use of a rapid exchange system is not limited to catheters; it is contemplated that an access sheath can also benefit from that which is disclosed herein.

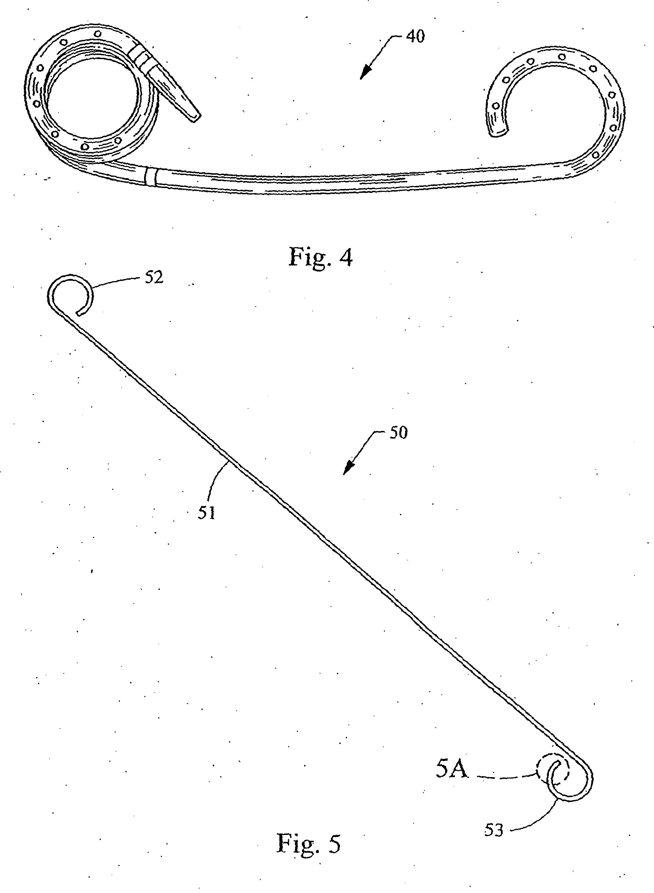

The access sheath may also be used to place a ureteral stent when the above diagnostic or therapeutic procedures are completed. No matter how gentle the procedures described above, there is a chance of some amount of trauma to the ureter during the procedures. Accordingly, it may be prudent to place a stent into the ureter to maintain patency of the ureteral lumen. Ureteral stents may be of the "double pigtail" variety, such as those available from Cook Urological Incorporated, Spencer, Indiana. Fig. 4 depicts one such stent 40. These ureteral stents are typically available in sizes of 4 Fr. to 8 Fr, and may be placed into a ureter using a wire guide and the procedure described above.

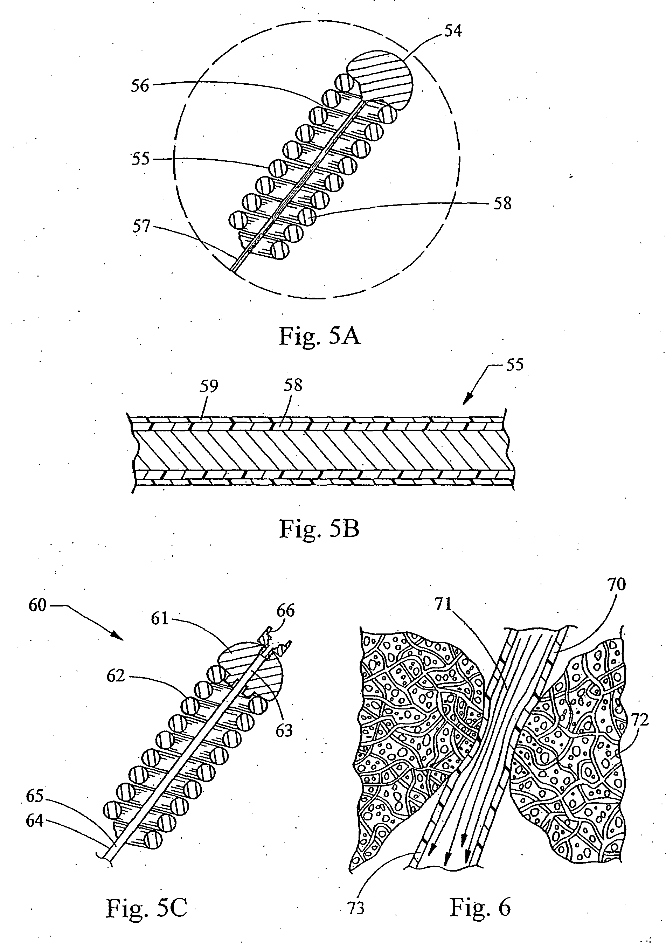

The procedure described above for dual, coaxial dilatation may be especially useful when there is a stricture or narrowing of a ureter for any reason. Fig. 6 depicts one such case. In Fig. 6, ureter 70 is constrained from its normal width 73 into a narrower path 71 along part of its length by a constricting body mass 72. An example would be a cancerous growth near the ureter that would cause compression on the ureter, e.g., colon cancer, bladder cancer, ovarian cancer, endometrial cancer, cervical cancer, and the like. In such cases, a stent with greater radial strength may be needed in order to maintain its lumen and allow drainage of urine through the ureter. Instead of elastomeric or plastic stents, a stent made from material that is more resistant to deformation may be needed. In addition, the stent must be removable without significant deformation or resistance.

Such a stent is depicted in Figs. 5, 5a, and 5b. Stent 50 is made from coiled wire along its length 51 and at both distal and proximal ends 52, 53, which may be substantially the same or may be different. The coils should be closely spaced so that they touch, but still allow fluid, such as urine or bile, to flow through the coils. The coils should also be spaced closely enough so that no tissue in growth occurs. Materials used in these stents are preferably biocompatible and corrosion-resistant. The wire is preferably made from alloys with minimal or low magnetic properties to avoid interference with diagnostic equipment, such as MRI machines. Alloys such as MP35N, MP 159, Astroloy M, Inconel 625, 316 stainless steel, 35N LT, Biodur 108, pure titanium, and Hastelloy S are preferred.

An inner wire 57 extends throughout the length of the stent and is secured to both ends 52, 53, such as by welding, brazing or swaging to a tip 54 on each end. The tips and the wire are preferably made from the same metallic alloy as the coil. The tips may be formed into a molten domed mass from the coiled wire and the inner wire during the joining process. It is important that both ends be atraumatic to the patient. The coils 55 have small gaps 56 between them so that urine may soak or leak into the stent in the kidney area or anywhere along the ureter and may leak out of the coils in the ureter or bladder area. The internal wire is helpful in preventing unraveling or extension of the coils, especially when the stent is being removed. The portion of the stent between the pigtails is preferably about 20 cm to about 32 cm long. Other lengths may be used. Suitable stents have a diameter of about the same as that of the catheter or pushing device. For example, a suitable stent could have a diameter of about 6 Fr., however, other diameters are contemplated depending on the needs of the patient and the procedure to be performed.

In order to present a surface highly resistant to encrustation during long-term implantation, stent embodiments should be highly polished, preferably electropolished. In electro-polishing, the article to be polished is placed into an electrolytic bath, but instead of being plated, the current is reversed. Asperities, tiny projections of metal on the surface of the stent coils, are vulnerable to this process, and are removed without changing the dimensions or temper of the stent. This highly polished surface is believed to be resistant to the bacteria responsible for encrustation because there are fewer sites with surface roughness suitable for adherence.

The wire 55 used for the outer coils is preferably coated, such as with a fluoropolymer or other protective, lubricious coating 58 before it is wound into a coil. It is preferred that the entire coil length be coated, while preserving the small gaps between the coil-turns of the stent for functioning of the stent drainage mechanism. In addition, a layer 59 of a preventive or other medication may be applied over coating 58, such as a layer containing heparin or other drug. Heparin tends to resist encrustation with long-term implantation of urinary tract medical devices. Heparin or other drug-containing coatings are preferably applied after the coil is wound. Fluoropolymers such as PTFE help to enable the bonding of certain drugs, such as heparin, to the surface of the coils and are therefore desirable in stents intended for long-term implantation. Other drugs useful for discouraging encrustation include heparin, covalent heparin, dexamethazone, dexamethasone sodium phosphate, dexamethasone acetate and other dexamethasone derivatives, triclosan, silver nitrate, ofloxacin, ciproflaxin, phosphorylcholine and triemethoprim.

In one preferred embodiment, the wire for coiling is coated, as by extrusion, with a fluoropolymer or other lubricious polymer or plastic material, and is then wound into a coiled stent, complete with end caps and a coated internal wire. The stent is then immersed into a solution of heparin, and a partial vacuum is applied to the vessel containing the solution. Preferred is a vacuum of about 10 Torr for a time period of about one minute to one hour, depending on the amount of coating desired. The stents are then rinsed in distilled water and dried before being packaged.

Another embodiment of a stent with greater radial strength is depicted in Fig. 5c. In this embodiment, which is similar to the embodiment of Fig. 5a, a narrow hollow cannula 64 extends between the distal and proximal ends of the stent 60. Stent 60 includes metallic ends 61 which include an orifice 63 to accommodate cannula 64. The stent includes a hollow outer coil 62 for greater radial strength. Cannula lumen 65 may be used to enable placement by a wire guide, and also may act as a lumen for drainage of body fluids, such as urine or bile. Fluid connector 66 may be attached to a proximal end of the cannula for connection for fluid drainage or for infusion of diagnostic or therapeutic fluids. The fluid connector may be attached by threads, by soldering, or brazing, or by any convenient method.

In addition, one or more additional medications or drugs may be placed on the surface of the stent in order to assist in patient care and comfort. For instance, an antimicrobial drug, such as a combination of rifampin and minocycline, may help to reduce inflammation and microbial activity in the vicinity of the stent. Antimicrobial coatings applied to the stent may include the following drugs, or their salts or derivatives: rifampin, minocycline, a mixture of rifampin and minocycline, a non-steroidal anti-inflammatory agent, a penicillin, a cephalosporin, a carbepenem, a betalactam, an antibiotic, an aminoglycoside, a macrolide, a lincosamide, a glycopeptide, a tetracyline, a chloramphenicol, a quinolone, a fucidin, a sulfonamide, a trimethoprim, a rifamycin, an oxaline, a streptogramin, a lipopeptide, a ketolide, a polyene, an azole, an echinocandin, alpha-terpineol, methylisothiazolone, cetylpyridinium chloride, chloroxyleneol, hexachlorophene, chlorhexidine and other cationic biguanides, methylene chloride, iodine and iodophores, triclosan, taurinamides, nitrofurantoin, methenamine, aldehydes, azylic acid, rifampycin, silver, benzyl peroxide, alcohols, and carboxylic acids and salts, and silver sulfadiazine. Also useful as antimicrobials are anthracyclines, such as doxorubicin or mitoxantrone, fluoropyrimidines such as 5-fluoroacil, and also podophylotoxins, such as etoposide. The salts and the derivatives of all of these are meant to be included as examples of antimicrobial drugs.

Analgesics, such as aspirin or other non-steroidal anti-inflammatory drugs, may also be applied to the surface to reduce pain and swelling upon implantation of the stent. These drugs or their salts or derivatives may include aspirin and non-steroidal anti-inflammatory drugs, including naproxen, choline, diflunisal, salsalate, fenoprofen, flurbiprofen, ketoprofen, ibuprofen, oxaprozin, diclofenac, indomethacin, sulindac, acetoaminophen, tolmetin, meloxicam, piroxicam, meclofenamate, mefanimic acid, nabumetone, etodelac, keterolac, celecoxib, valdecoxib and rofecoxib, mixtures thereof, and derivatives thereof.

Other analgesics or anesthetics that may be coated onto the surface of the stent include opioids, synthetic drugs with narcotic properties, and local anesthetics to include at least paracetamol, bupivacaine, ropivacaine, lidocaine, and novacaine.alfentanil, buprenorphine, carfentanil, codeine, codeinone, dextropropoxyphene, dihydrocodeine, endorphin, fentanyl, hydrocodone, hydromorphone, methadone, morphine, morphinone, oxycodone, oxymorphone, pethidine, remifantanil, sulfentanil, thebaine, and tramadol, mixtures thereof, and derivatives thereof.

Any of these drugs and coatings are preferably applied in a time-release manner so that the beneficial effect of the drug is sustained over a period of at least several weeks or months. This may be especially helpful in the case where a stent or catheter will remain in place for a considerable length of time.

While the present dilation technique is highly useful for placement of ureteral stents, the technique may also be used for placing stents and catheters for use in other hollow parts or blood vessels of the body. These may include biliary or gall bladder stents, stents for use in percutaneous nephrostomy procedures, hepatic drainage, gastrointestinal drainage, and so on, for drainage of other body cavities. It is intended that the foregoing detailed description be regarded as illustrative rather than limiting, and that it be understood that it is the following claims, including all equivalents, that are intended to define the scope of this invention. Furthermore, the foregoing description are not intended to limit the scope of the invention described herein or with regard to the details of its construction and manner of operation. It will be evident to one skilled in the art that modifications and variations may be made without departing from the scope of the invention. Changes in form and in the proportion of parts, as well as the substitution of equivalents, are contemplated as circumstances may suggest and render expedience; although specific terms have been employed, they are intended in a generic and descriptive sense only and not for the purpose of limiting the scope of the invention set forth in the following claims.

## Claims

1. An access kit comprising:
a stent (50);
a catheter (32) having a proximal portion and a distal portion;
an access sheath (33) having a proximal portion and a distal portion;
wherein the catheter is removably connected to the access sheath;
the catheter and the access sheath are substantially coaxial;
the catheter is longer than the access sheath; and
the length of the catheter is less than twice the length of the access sheath **characterised in that** the stent is deployable through the access sheath by a stent positioner; and wherein the catheter optionally serves as the stent positioner.

2. The kit of Claim 1, wherein the access sheath is about 70 cm in length.

3. The kit of Claim 1, wherein the catheter is about 50-85 cm in length.

4. The kit of Claim 1, wherein the catheter and the access sheath are configured for use with a wire guide having a length of about 145-150 cm.

5. The kit of Claim 1, wherein the proximal portion of the catheter further comprises a connector;
wherein the proximal portion of the access sheath further comprises a connector; and
wherein the connector of the catheter is configured to removably connect to the connector of the access sheath.

6. The kit of Claim 1, wherein at least one of the catheter and the access sheath further comprises at least one marking band observable with an x-ray or fluoroscopic.

7. The kit of Claim 1, wherein at least one of the catheter and access sheath is a rapid exchange system.

8. The kit of Claim 1, wherein the stent and access sheath are each configured to be inserted in a ureter.

9. The kit of Claim 1, wherein the catheter is configured to receive an injected fluid therethrough.

10. The kit of Claim 9, wherein the catheter further comprises a female Luer lock fitting configured to inject fluid into the catheter.

11. The kit of Claim 1, wherein the stent is metallic.

12. The kit of Claim 1, wherein the stent is a double pigtail ureteral stent.

13. The kit of Claim 1, wherein the stent comprises a hollow coiled wire, the coils of the coiled wire are configured to be closely spaced so that they touch, but allow fluid to flow through the coils, and an inner wire extending through the length of the stent and secured to an atraumatic end on the respective end of the stent, the wire being secured to an atraumatic end cap on each opposite end of the stent.

14. The kit of Claim 13, wherein the opposite ends of the stent each comprises a pigtail portion.

## Patentansprüche

1. Zugangskit, das Folgendes umfasst: einen Stent (50); einen Katheter (32) mit einem proximalen Abschnitt und einem distalen Abschnitt; einer Zugangsschleuse (33) mit einem proximalen Abschnitt und einem distalen Abschnitt; worin der Katheter entfernbar mit der Zugangsschleuse verbunden ist; wobei der Katheter und die Zugangsschleuse im Wesentlichen koaxial zueinander sind; wobei der Katheter länger ist als die Zugangsschleuse; und wobei die Länge des Katheters weniger als die doppelte Länge der Zugangsschleuse beträgt; **dadurch gekennzeichnet, dass** der Stent von einem Stentpositionierer durch die Zugangsschleuse eingeführt werden kann; und worin der Katheter fakultativ als Stentpositionierer dient.

2. Kit nach Anspruch 1, worin die Zugangsschleuse ca. 70 cm lang ist.

3. Kit nach Anspruch 1, worin der Katheter ca. 50-85 cm lang ist.

4. Kit nach Anspruch 1, worin der Katheter und die Zugangsschleuse zusammen mit einem Führungsdraht mit einer Länge von ca. 145-150 cm verwendet werden können.

5. Kit nach Anspruch 1, worin der proximale Abschnitt des Katheters ferner ein Verbindungsglied umfasst; worin der proximale Abschnitt der Zugangsschleuse ferner ein Verbindungsglied umfasst; und worin das Verbindungsglied des Katheters entfernbar mit dem Verbindungsglied der Zugangsschleuse verbunden werden kann.

6. Kit nach Anspruch 1, worin zumindest der Katheter und/oder zumindest die Zugangsschleuse ferner zumindest ein Markierungsband umfasst, das unter Röntgenstrahlung oder fluoroskopisch sichtbar ist.

7. Kit nach Anspruch 1, worin zumindest der Katheter und/oder zumindest die Zugangsschleuse ein schnell auswechselbares System ist.

8. Kit nach Anspruch 1, worin der Stent und die Zugangsschleuse jeweils in einen Harnleiter eingeführt werden können.

9. Kit nach Anspruch 1, worin der Katheter eine Injektionsflüssigkeit durch ihn hindurch aufnehmen kann.

10. Kit nach Anspruch 9, worin der Katheter ferner eine Luer-Lock-Aufnahmevorrichtung zur Injektion von Flüssigkeit in den Katheter umfasst.

11. Kit nach Anspruch 1, worin der Stent aus Metall besteht.

12. Kit nach Anspruch 1, worin der Stent ein Double-Pigtail-Harnleiterstent ist.

13. Kit nach Anspruch 1, worin der Stent einen hohlen aufgerollten Draht, wobei die Spulen des aufgerollten Drahts im engen Abstand zueinander liegen, so dass sie sich berühren, aber Flüssigkeitsfluss durch die Spulen zulassen, und einen sich durch die Länge des Stents erstreckenden und an einem atraumatischen Ende am jeweiligen Ende des Stents befestigten Innendraht umfasst, wobei der Draht an einer atraumatischen Endkappe an jedem gegenüberliegenden Ende des Stents befestigt ist.

14. Kit nach Anspruch 13, worin die gegenüberliegenden Enden des Stents jeweils einen Pigtail-Abschnitt umfassen.

## Revendications

1. Kit d'accès, comprenant:
un stent (50);
un cathéter (32) présentant une partie proximale et une partie distale;
une gaine d'accès (33) présentant une partie proximale et une partie distale;
dans lequel le cathéter est connecté de façon détachable à la gaine d'accès;
le cathéter et la gaine d'accès sont sensiblement coaxiaux;
le cathéter est plus long que la gaine d'accès; et
la longueur du cathéter est plus petite que deux fois la longueur de la gaine d'accès,
**caractérisé en ce que** le stent peut être déployé à travers la gaine d'accès par un positionneur de stent, et
dans lequel le cathéter sert optionnellement de positionneur de stent.

2. Kit selon la revendication 1, dans lequel la gaine d'accès présente une longueur d'environ 70 cm.

3. Kit selon la revendication 1, dans lequel le cathéter présente une longueur d'environ 50 cm à 85 cm.

4. Kit selon la revendication 1, dans lequel le cathéter et la gaine d'accès sont configurés de manière à être utilisés avec un guide-fil dont la longueur est d'environ 145 cm à 150 cm.

5. Kit selon la revendication 1, dans lequel la partie proximale du cathéter comprend en outre un connecteur;
dans lequel la partie proximale de la gaine d'accès comprend en outre un connecteur; et
dans lequel le connecteur du cathéter est configuré de manière à se connecter de façon détachable au connecteur de la gaine d'accès.

6. Kit selon la revendication 1, dans lequel au moins soit le cathéter soit la gaine d'accès comprend en outre au moins une bande de marquage que l'on peut observer aux rayons X ou par fluoroscopie.

7. Kit selon la revendication 1, dans lequel au moins soit le cathéter soit la gaine d'accès est un système à échange rapide.

8. Kit selon la revendication 1, dans lequel le stent et la gaine d'accès sont chacun configurés de manière à être insérés dans un uretère.

9. Kit selon la revendication 1, dans lequel le cathéter est configuré de manière à recevoir un fluide qui est injecté à travers lui.

10. Kit selon la revendication 9, dans lequel le cathéter comprend en outre un embout Luer à verrouillage femelle qui est configuré pour injecter un fluide dans le cathéter.

11. Kit selon la revendication 1, dans lequel le stent est métallique.

12. Kit selon la revendication 1, dans lequel le stent est un stent urétéral en double queue de cochon.

13. Kit selon la revendication 1, dans lequel le stent comprend un fil hélicoïdal creux, les spires du fil hélicoïdal étant configurées de manière à être étroitement espacées de telle sorte qu'elles se touchent, mais permettent à un fluide de s'écouler à travers les spires, et un fil intérieur qui s'étend à travers la longueur du stent et qui est fixé à une extrémité atraumatique sur l'extrémité respective du stent, le fil étant fixé à une coiffe d'extrémité atraumatique sur chaque extrémité opposée du stent.

14. Kit selon la revendication 13, dans lequel les extrémités opposées du stent comprennent chacune une partie en queue de cochon.
